# EUROPEAN PATENT SPECIFICATION

(11) **EP 2 550 993 B1**
(45) Date of publication and mention of the grant of the patent: **10.12.2014**
(21) Application number: 11193913.8
(22) Date of filing: 16.12.2011
(51) Int. Cl.: A61N 5/06

(54) **Devicefor altering dopamine Level**
Vorrichtung zur Veränderung des Dopaminspiegels
Dispositif d'altération de niveau de dopamine

(30) Priority: 29.03.2011 FI 20115295; 29.03.2011 US 201113074230
(43) Date of publication of application: 30.01.2013
(73) Proprietor: Valkee Oy, 90590 Oulu (FI)
(72) Inventor: Nissilä, Juuso, 91100 Ii (FI); Aunio, Antti, 90410 Oulu (FI)
(74) Representative: Høiberg A/S

(56) References cited:
- WO-A1-00/24465
- WO-A1-2008/029001
- WO-A2-2006/138659
- WO-A2-2008/016894

## Description

The present invention relates to a device for non-invasively applying optical radiation to photosensitive parts of the brain.

### Background of invention

It is well known that light has a direct effect on human health because of the way it influences the circadian rhythm. And the human (and animal) brain includes regions that can be affected by optical radiation. This may result in a metabolic or nervous responsive stimulation, which may appear as a change in concentrations of several hormones and neuro-transmitters. The light may be natural sunlight but also artificial light sources are known to have an effect. The hormone melatonin is secreted in the pineal gland as a response to light and helps to control the circadian rhythm. Secretion of melatonin peaks at night and ebbs during the day and its presence provides information about night-length. Disruption of the circadian rhythm, e.g. due to lack of daylight or crossing time zones, usually has a negative effect and may lead to jet lag, bipolar disorders and circadian rhythm sleep disorders such as seasonal affective disorder (SAD) and delayed sleep phase syndrome (DSPS). In addition most mood disorders and many neurological disorders have a pattern of symptomality changing in accordance to annual rhythm.

It is typically necessary to use artificial optical radiation when natural light is not sufficient for achieving a desired physiological effect. Artificial optical radiation may be generated by bright light therapy devices in the form of the well-known bright light lamps and recently a more elegant solution has been commercialized in the form of the Valkee Brain Stimulation Headset described in WO 2008/029001 wherein bright light LED (Light Emitting Diode) light is provided to the brain through the auditory canal.

### Summary of invention

In general the present invention relates to a device for non-invasive light therapy comprising one or more radiation units adapted to direct optical radiation at one or more neuroanatomical brain structures from an extra-cranial position of a user. In order to avoid absorption in the cerebrum the device and/or the radiation unit(s) may be adapted to direct the optical radiation from at least one extra-cranial position below the cerebrum of the user. Said at least one extra-cranial position is preferably non-ocular, i.e. the optical radiation is preferably not provided through the eyes of the user. The device may comprise a housing in optical and/or electrical connection with said one or more radiation units. Further, the device may comprise one or more light sources, e.g. LED's, for generating the optical radiation. The device may comprise means for adapting the intensity of the optical radiation, such as electronic control means, e.g. situated in the housing, for adjusting the power supplied to the light sources. The device may further comprise means for adapting the spectral composition of the optical radiation. For some types of light sources the spectral composition of the emitted light may be changed by varying the temperature or power of applying different types of filters or phosphorizing elements in the light path. The spectral composition may also be varied by combining different light sources with different spectral compositions and independently controlling the different light sources. The optical radiation emitted from a radiation unit may be applied with a predefined setting, e.g. the optical radiation (continuous wave (CW) or pulsed) may be applied with one or more of the following parameters being predefined: duration, intensity, pulse frequency, total power and spectral composition.

The invention is defined in the claims. Other embodiments are exemplary.

### Description of drawings

- Fig. 1a: is a cross-sectional side view illustration of the human brain pointing out different neuro-anatomical brain structures and serotonin pathways.
- Fig. 1b: shows a cross-sectional schematic diagram of the human brain illustrating specific neuro-anatomical regions and serotonin pathways.
- Fig. 2: shows normalized absorption spectra of various opsins.
- Fig. 3: shows measured relative amounts of OPN3 and OPN4 in different regions of the human brain.
- Fig. 4: is a cross-sectional side view illustration of the human brain showing the dopamine pathways.
- Fig. 5: shows an illustration of how light can be distributed to photosensitive regions in the brain via the auditory canal.

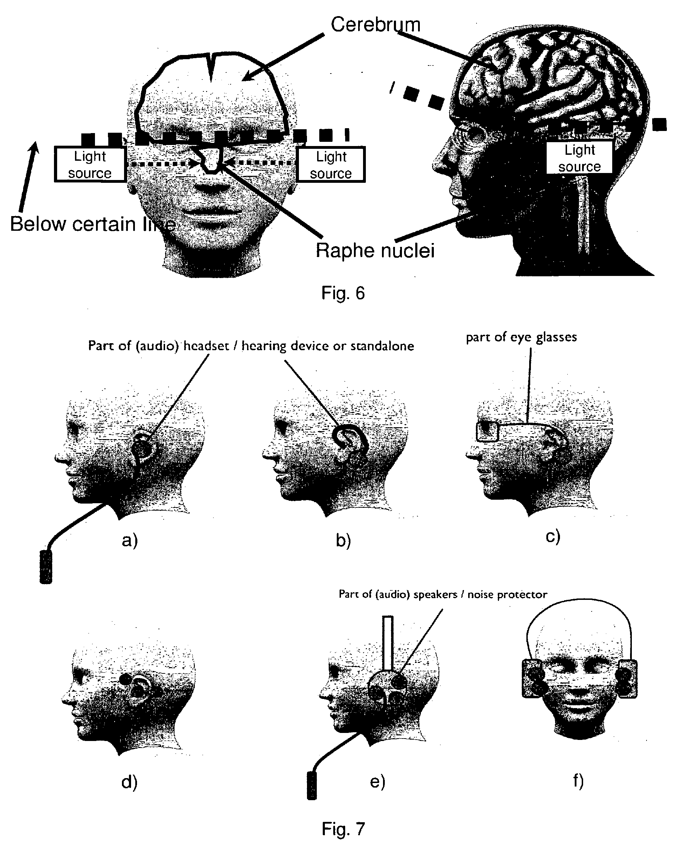

- Fig. 6: show frontal and cross-sectional side view illustrations of a human head where the size of the cerebrum is indicated.
- Fig. 7a)-f): show possible placements and different embodiments of the device according to the invention.
- Fig. 8a: shows an exemplary spectral composition of a white LED.
- Fig. 8b: shows the spectral LED composition of fig. 8a where the spectral output in three different wavelength intervals are indicated.
- Fig. 8c: shows the spectral composition of four different LEDs and their combined spectral composition.
- Fig. 9: shows top and side view schematic illustrations of an example of a (part of a) radiation unit with four different light sources.
- Fig. 10: is a schematic illustration of a high level architecture of a network system incorporating a device according to the invention.
- Fig. 11: shows three different spectral compositions, each having two intensity peaks but with different ratios of spectral outputs integrated around each peak.
- Fig. 12: shows the procedure of a controlled dose-response study of transcranial light therapy disclosed in example 1.
- Fig. 13: shows response rates measured by SIGH-SAD, HAMA and BDI in different treatment groups in the light therapy dose-response study.
- Fig. 14: shows BDI sum scores in different treatment groups during the four-week treatment period of the light therapy dose-response study.
- Figs. 15A-E: show various spectral compositions of LED's of certain types.
- Figs. 16A-B: show measured progress for type A and type B trail making test for test persons which have been subject to trans-cranial light therapy (see examples 6 and 7).
- Figs. 17A-D: show measured reaction time to auditory and visual stimulus for two groups of test persons (see example 8).
- Figs. 18A-B: show the improvement in reaction time for the two groups (see example 8).

### Definitions

**Disease** as used herein is an abnormal condition of an organism that impairs bodily functions, associated with specific symptoms and signs, typically causes discomfort and/or dysfunction. Herein used interchangeably with disorder.

**Disorder** as used herein is a functional abnormality or disturbance. Herein used interchangeably with disease

**Opsins** (OPN) are light-sensing proteins belonging to the superfamily of G-protein-coupled receptors (GPCR). Opsins are known to mediate phototransduction in both visual and nonvisual systems by being transmembrane receptorproteins. Main principle of action for these receptors is to activate a guanine nucleotide binding protein (Gprotein) and an effector enzyme to produce response in the attached cell. Based on sequence homology, opsins can be distinguished in to six subfamilies: vertebrate opsin/encephalopsin, Go, Gs, Gq, photoisomerase and neuropsin subfamilies. On the basis of cellular expression/function and phylogeny, the classification has three categories - ciliary opsins, rhabdomeric opsins and photoisomerases. According to the name, ciliary opsins are thought to localise in ciliary photoreceptor cells, rhabdomeric opsins in rhabdomeric photoreceptor cells, whereas photoisomerases are a group of opsins sharing same phylogeny and intron positions.

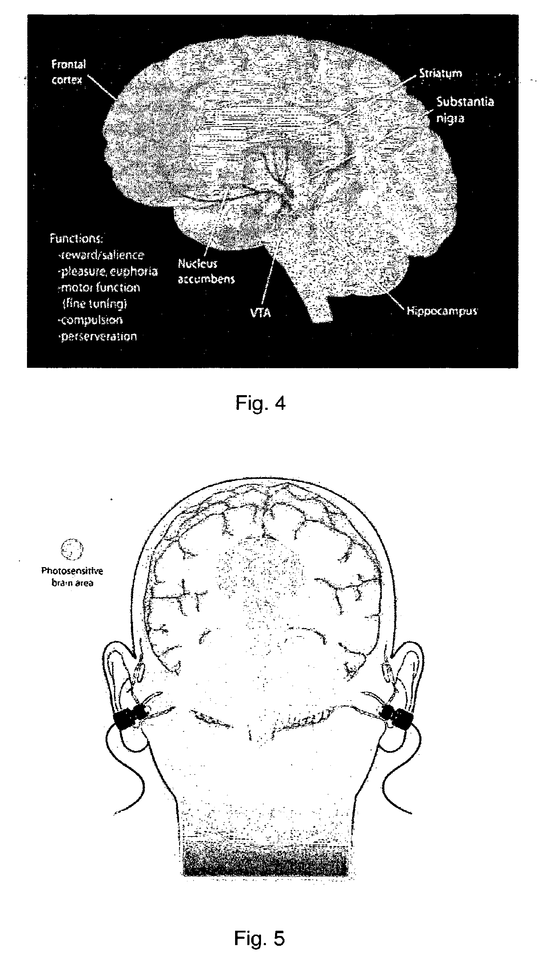

Fig. 4 shows measured relative amounts of OPN3 (left column) and OPN4 (right column) in different neuro-anatomical structures / regions of the human brain.

**Table 1: Chromosomal locations and number of introns of the nine human opsin genes.**

| Opsin | Chromosomal location | Number of introns |
|---|---|---|
| Rhodopsin | 3q22.1 | 4 |
| Blue opsin | 7q32.1 | 4 |
| Red opsin | Xq28 | 5 |
| Green opsin | Xq28 | 5 |
| Encephalopsin | 1q43 | 3 |
| Melanopsin | 10q23.2 | 9 |
| Peropsin | 4q25 | 6 |
| RGR | 10q23.1 | 6 |
| Neuropsin | 6p12.3 | 6 |

**Phototransduction** is a process by which light is converted into electrical signals in the rod cells, cone cells and photosensitive ganglion cells of the retina of the eye. In general, all known vertebrate photoreceptors use an opsin protein bound to a vitamin A-chromophore as photopigment. Over species and opsins, the principle of phototransduction is always the same: When the photon is absorbed by the retinal chromophore, this molecule isomerizes from 11-cis-retinal form to the all-trans-retinal form. This conformational change allows opsin-proteins intracellular terminus to trigger a G-protein cascade leading into rise in receptor membrane potential. Phototransduction is this cascade converting photic energy into neural responses. In addition phototransduction may cover also reactions on receptors and chemical compounds called excitatory aminoacids (e.g. glutamate receptor), when said receptors may have allosterical regulation due to illumination. This phenomena at least partly overlaps and coincides the opsin-mediated phototransduction and may also participate to the release of neurotransmitters, monoamines, psychogenic amines, GABA, NMDA and several other messengers affecting to the signal transduction in the brain and elsewhere in the CNS. Conceptually, the same cascade, as in the eye, happens on the neural cells of the brain when opsins are absorbing photons. In addition in this document phototransduction mostly refers to relatively slow and modulatory changes in the membrane potential.

### Diseases and disorders relating to chemical imbalances

### Mental diseases

A mental disorder or mental illness is a psychological or behavioral pattern that occurs in an individual and is thought to cause distress or disability that is not expected as part of normal development or culture. For example a depressed mood is often reported as feeling sad, helpless, and hopeless.

### Parkinson's disease

(also known as Parkinson disease, Parkinson's, idiopathic parkinsonism, primary parkinsonism, PD, or paralysis agitans) is a degenerative disorder of the central nervous system. The motor symptoms of Parkinson's disease result from the death of dopamine-generating cells in the substantia nigra. The aetiology is considered to be different mechanisms of cellular damage in substantia nigra. The damage might be related to cell death caused by inflammation. The inflammation and cell death may also be caused by autoimmune routes, also different heavy-metal poisonings might be the reason for autolysis of substantia nigra regions, having immunoreaction central in its mechanism.

A particular conceptual model of the motor circuit and its alteration with Parkinson's disease has been of great influence. In this model, the basal ganglia normally exert a constant inhibitory influence on a wide range of motor systems, preventing them from becoming active at inappropriate times. When a decision is made to perform a particular action, inhibition is reduced for the required motor system, thereby releasing it for activation. Dopamine acts to facilitate this release of inhibition, so high levels of dopamine function tend to promote motor activity, while low levels of dopamine function, such as occur in Parkinson's disease, demands greater exertions of effort for any given movement. Thus the net effect of dopamine depletion is to produce hypokinesia, an overall reduction in motor output. There is presently no cure for Parkinson's disease, but medications, surgery and multidisciplinary management can provide relief from the symptoms.

The main families of drugs useful for treating motor symptoms are levodopa, dopamine agonists and MAO-B inhibitors. Levodopa has been the most widely used treatment for over 30 years. Since motor symptoms are produced by a lack of dopamine in the substantia nigra, the administration of Levodopa temporarily diminishes the motor symptoms. Levodopa is converted into dopamine in the dopaminergic neurons by dopa decarboxylase but only 5-10% of Levodopa actually crosses the blood-brain barrier. The remainder is often metabolized to dopamine elsewhere, thus possibly producing excessive dopamine activity, allowing motor systems to be activated at inappropriate times and thereby producing dyskinesias, marked by involuntary writhing movements, and other side effects including nausea and joint stiffness. The drug based treatments are effective at managing the early motor symptoms of the disease. As the disease progresses and dopamine neurons continue to be lost, a point eventually arrives at which these drugs become ineffective at treating the symptoms and at the same time produce dyskinesia.

Tobacco smokers' risk of having Parkinson's disease may be reduced down to a third when compared to non-smokers. The basis for this effect is not known, but possibilities include an effect of nicotine as a dopamine stimulant.

### Obsessive-compulsive disorder (OCD)

is an anxiety disorder characterized by intrusive thoughts that produce uneasiness, apprehension, fear, or worry, by repetitive behaviours aimed at reducing the associated anxiety, or by a combination of such obsessions and compulsions. Imbalance of brain chemicals, especially serotonin and dopamine, may contribute to OCD. Independent studies have consistently found unusual dopamine and serotonin activity in various regions of the brain in individuals with OCD. These can be defined as dopaminergic hyperfunction in the prefrontal cortex and serotonergic hypofunction in the basal ganglia.

### Alcohol addiction

*Alcoholism* is a broad term for problems with alcohol, and is generally used to mean compulsive and uncontrolled consumption of alcoholic beverages. It is medically considered a neurological disorder. Alcohol causes the body to release endorphins, which in turn release dopamine and activate the reward pathways.

### Tobacco and nicotine addiction

When a cigarette is smoked, nicotine-rich blood passes from the lungs to the brain within seven seconds and immediately stimulates the release of many chemical messengers including acetylcholine, norepinephrine, epinephrine, vasopressin, arginine, dopamine, autocrine agents, and beta-endorphin. This release of neurotransmitters and hormones is responsible for most of nicotine's effects. Nicotine also extends the duration of positive effects of dopamine and increases sensitivity in brain reward systems. Modern research shows that nicotine acts on the brain to produce a number of effects. Specifically, research examining its addictive nature has been found to show that nicotine activates the Mesolimbic pathway ("reward system") - the circuitry within the brain that regulates feelings of pleasure and euphoria. By increasing the levels of dopamine within the reward circuits in the brain, nicotine acts as a chemical with intense addictive qualities. Like other physically addictive drugs, nicotine withdrawal causes down-regulation of the production of dopamine and other stimulatory neurotransmitters as the brain attempts to compensate for artificial stimulation.

### Detailed description of invention

### Device

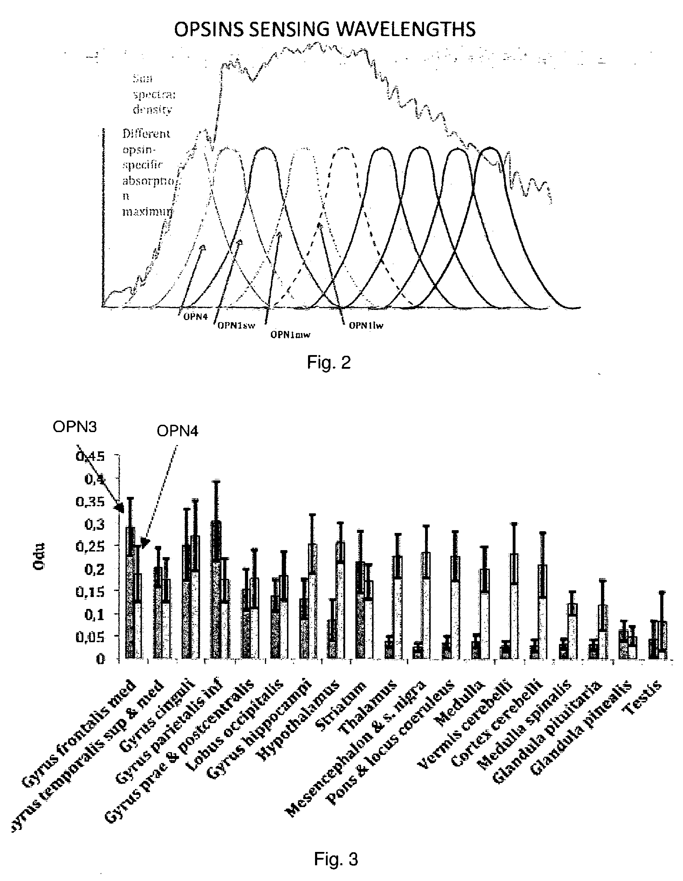

By conducting experiments on preserved human brains donated from deceased subjects the inventors have been able to measure the presence and amount of various light sensitive opsins in different parts of the human brain. The inventors have thereby realized that because the relative and absolute amounts of specific light sensitive opsins vary in different neuro-anatomical structures of the human brain the reaction in and response of a specific neuro-anatomical brain structure when illuminated with optical radiation is depending on the nature of the radiation, i.e. the nature of the light has a marked influence on the reaction / stimulation pattern in the brain. In particular the spectral composition of the light seems to be important, because as the example illustrated in fig. 3 for OPN3 and OPN4 the absolute and relative amounts of OPN3 and OPN4 vary for the different brain structures, and as OPN3 and OPN4 has different absorption wavelengths the reaction in a specific brain region will depend on wavelength composition of the light. A first aspect of the invention therefore relates to a device for non-invasive light therapy comprising one or more radiation units adapted to direct optical radiation at one or more neuroanatomical brain structures of a user through at least one auditory channel of the user, wherein the optical radiation emitted from a radiation unit is applied with one or more of the following parameters being predefined for a plurality of predefined wavelength intervals: duration, intensity, total power, spectral output and spectral composition, and wherein the optical radiation emitted from a radiation unit is applied with a predefined ratio of: a first spectral output integrated over a first wavelength interval, and a second spectral output integrated over a second wavelength interval.

Natural bright sunlight at noon (about 80,000-150,000 lux) far exceeds the amount which is normally emitted by conventional bright light lamps (2,500 - 10,000 lux). The relatively low illuminations derived from conventional light devices reach deep structures of the brain mainly visually, but only to some extent by penetrating via other routes. An advantage of the device according to the present invention is that the photic energy penetrating the skull far exceeds the amount which is normally visibly tolerable and emitted by conventional bright light lamps.

The optical radiation emitted from a radiation unit is applied with predefined ratios of: a first spectral output integrated over a first wavelength interval, a second spectral output integrated over a second wavelength interval, and a third spectral output integrated over a third wavelength interval.

In one embodiment of the invention the spectral composition of the optical radiation is adapted to the absorption spectrum of one or more light sensitive opsins. Preferably at least one of said opsins is present in at least one of said neuro-anatomical structures.

In one embodiment of the invention the optical radiation comprises light from a plurality of wavelength intervals. E.g. if the light emitted from a radiation unit is composed of light from different light sources with different wavelength characteristics. Preferably means for adapting the intensity, timing, total power and/or total energy of optical radiation provided in each wavelength interval are then provided. And further means for adapting the ratio of the intensity, timing, total power and/or total energy of optical radiation provided in the wavelength intervals are also preferably provided.

The invention the optical radiation comprises light in a first wavelength interval with a start point of 410 nm and an endpoint of 480 nm.

The invention the optical radiation comprises light in a second wavelength interval with a start point of 485 nm and an endpoint of 700 nm.

The invention the optical radiation comprises light with a predefined ratio of a first spectral output in a first wavelength interval and a second spectral output in a second wavelength interval. Said predefined ratio is between 0.65.

In one embodiment of the invention the optical radiation is generated by a plurality of different light sources, such as a plurality of light sources with different spectral characteristics. The spectral composition of the optical radiation emitted from the radiation unit(s) can then be adapted by controlling the light sources independently.

In a further embodiment of the invention the radiation unit comprises one or more elements and/or layers of one or more phosphorizing compounds. The principle of a white LED may be used for creating different spectral characteristics. E.g. one or more LED's may be arranged behind different phosphorizing layers, the resulting spectrum is depending on the nature (e.g. composition, thickness, etc.) of the specific phosphorizing layer.

Light sources for generating the optical radiation may be accommodated in a housing of the device. With an optical connection to the radiation unit(s) the light may be distributed for illumination of the user.

In another embodiment of the invention one or more light sources are accommodated in each radiation unit.

In yet another embodiment of the invention each radiation unit comprises a single light source. If there is a plurality of radiation units, each having a single light source, there may be a plurality of different light sources with different wavelength characteristics. The specific single light source is preferably chosen to have a spectral characteristic suitable for one or more specific medical treatments. The radiation units may then be "paired", i.e. there may be a plurality of paired radiation unit having the same type of light source. Then there can be a pair of radiation units for different applications.

In one embodiment of the invention at least one radiation unit comprises a light guide for guiding at least a part of said optical radiation. A light source, such as an LED, emits light in many directions. A light guide may collect some of this light and direct it in a certain direction.

In another embodiment of the invention at least one radiation unit is adapted to be arranged at an external ear of the user, e.g. by using the ear as attachment "peg". The earlobe may also be used, i.e. a radiation unit may be adapted to be arranged on or via an earlobe of the user.

As known from the prior art device at least one radiation unit may be adapted to be arranged at least partly inside an external auditory canal of the user. At least one radiation unit may be adapted to be arranged such that at least part of said optical radiation is guided into an external auditory canal of the user.

In a further embodiment of the invention the radiation units may be arranged via a headband structure accommodating the radiation unit(s).

In one embodiment of the invention said one or more neuroanatomical brain structures are selected from the group of: the brain stem, medulla oblongata, pons, midbrain, substantia nigra, raphe nuclei, nucleus raphe obscurus, raphe magnus, raphe pontis, raphe pallidus, nucleus centralis superior, nucleus raphe dorsalis, nuclei linearis intermedius and linearis rostralis.

The inventors have observed opsins (OPN3, OPN4 and most recently, rhodopsin) all over the brains. They are abundant at least in:
- frontal cortex
- temporal lobes
- cingutar cortex
- parietal lobe
- postcentral area and prae
- occipital lobe
- hippovampus
- hypothalamus
- striatum
- thalamus
- mesencephalon including substantia nigra
- pons incl. raphe nucleus
- medulla
- cerebellum (vermis)
- cerebellar cortex
- medulla spinalis
- Pituitary gland
- pineal gland

in addition also testis are photosensitive to at least via OPN3 & OPN4

The amount of possible diseases and symptoms via these regions is huge, including all major mood diseases, neurological, hormonal, cognitive, motor performance, memory & learning, language, skill or any brain related disease/symptom. Also, the wide variety of different wavelengths seem to have an array of responses due to different concentrations of each opsin in different brain areas.

### Dopamine

The inventors have realized that the non-invasive application of light therapy may trigger the production of dopamine in various parts of the human brain. Thus, a further aspect of the invention relates to a novel application of the device, i.e. a device for non-invasive light therapy comprising one or more radiation units adapted to direct optical radiation at one or more neuroanatomical brain structures, such as substantia nigra, said device applied for use in altering the level of dopamine in at least one of said one or more neuroanatomical brain structures. In particular the inventors have realized that the dopamine level (produced) in substantia nigra, substantia nigra pars compacta and the ventral tegmental area may be influenced by applied light.

Dopamine is a catecholamine neurotransmitter present in a wide variety of animals, including both vertebrates and invertebrates. In the brain, this substituted phenethylamine functions as a neurotransmitter, activating the five known types of dopamine receptors: D1, D2, D3, D4, and D5 and their variants. Dopamine is produced in several areas of the brain, including the substantia nigra and the ventral tegmental area. Dopamine has many functions in the brain, including important roles in behavior and cognition, voluntary movement, motivation, punishment and reward, inhibition of prolactin production (involved in lactation and sexual gratification), sleep, mood, attention, working memory, and learning.

Dopamine is available as intravenous medication acting on the sympathetic nervous system, producing effects such as increased heart rate and blood pressure. However, because dopamine cannot cross the blood-brain barrier, dopamine given as a drug does not directly affect the central nervous system. To increase the amount of dopamine in the brains of patients with diseases such as Parkinson's disease and dopa-responsive dystonia, L-DOPA (the precursor of dopamine) is often given because it crosses the blood-brain barrier relatively easily.

With the present invention the principal source of dopamine production can be targeted directly. This opens for a range of applications for a device delivering optical radiation. Thus, a further embodiment of the invention relates to a device for non-invasive light therapy comprising one or more radiation units adapted to direct optical radiation at one or more neuroanatomical brain structures, such as substantia nigra, of a user from at least one extra-cranial position below the cerebrum of the user, said device applied for use in treatment of Parkinson's disease, burning mouth syndrome, fibromyalgia, restless legs syndrome, social anxiety, ADHD and/or hypertension (HTN). The device may further be applied for use in pain processing in multiple levels of the central nervous system.

A further embodiment of the invention relates to a device adapted for treatment of a disorder comprising the step of non-invasively applying a physical stimulus in the form of optical radiation to a neuro-anatomical brain structure, such as substantia nigra, wherein the physical stimulus is sufficient for inducing a change in the level, such as an increase in the level, of dopamine in said neuro-anatomical brain structure, wherein said disorder is selected from the group of Parkinson's disease and hypertension (HTN). Also, light-induced allostery of EAA-receptors is involved in this, making the dopamine pathways work optimally when excretion and other dynamics of action are induced photically.

Affecting the dopamine system using light therapy has a potential effect to any known disease / disorder / symptoms having aetiology or underlying mechanisms relating to the dopamine system. More and more are found, but to date known are at least:
- Parkinsons
- anxiety
- depression
- cognitive impairment

### Treatment

The present invention provides the means for the prevention, treatment and/or amelioration of diseases and/or disorders which are related to chemical imbalances of the body, in particular chemical imbalances in the brain and in particular the human brain.

The processes that occur in the brain during radiation with light have not been completely outlined. It is known that opsins mediate phototransduction in both visual and non-visual systems by being trans-membrane proteins acting as G-protein-coupled receptors (GPCRs). Until now, several studies, per se, show that genes of extra-visual opsins are also expressed in mammalian brain in the mRNA-level. Furthermore, there is now initial evidence that e.g. encephalopsin, also termed OPN3 or panopsin exists in the protein level in the human brain. OPN3 has been suggested to play a role in non-visual photic processes such as the entrainment of circadian rhythm or the regulation of pineal melatonin production even though the exact function of encephalopsin is largely unknown. However, the role of OPN3 is most likely related into its phylogenetic background as an extra-retinal ciliary phototransductive membrane protein, e.g. by means on ciliary structure. OPN3 may also affect neuro regenerator and stem cell differentiation into neural and/or glial cells in the brain. OPN3 may also affect immune response and inflammation in the brain.

With the present invention it has been realized that endogenous opsins maintain their functional role as a part of extra-visual phototransduction. Consequently it is has been realized by the inventors that light therapy does not have to be mediated through the eyes.

It is an object of the present invention to target specific opsins in specific neuro-anatomical regions of the brain by means of non-invasive applied optical radiation. It is a further object of the present invention to thereby alter the amount, level and/or production of one or more hormones, neuro-transmitters or other chemical compound in said neuro-anatomical brain region, and thereby correct chemical imbalances in the body and/or brain which may be the cause of a disorder.

### Individual to be treated

The "user" of the device according to the present invention may be any animal or human being. The device is generally applied on the head of an animal or a human being. Preferably the individual to be treated is a mammal, such as but not limited to a dog, cat, primate, horse, cow or human being.

### Detailed description of Drawings

Pharmaceutical drugs are distributed to the body via the blood system. It is therefore likely that the drug will be distributed wherever the blood system reaches. It is further likely that this will cause a substantially equal increase of the "base level" concentration of the compound / hormone / neurotransmitter in the body. Adverse side effect of pharmaceuticals might arise due to this general increase in base level that might cause an imbalance compared to the natural concentration distribution of the compound / hormone / neurotransmitter. With the present device the natural production of the compound / hormone / neurotransmitter is targeted thereby maintaining the natural distribution channels that will lead to a correct distribution to target areas.

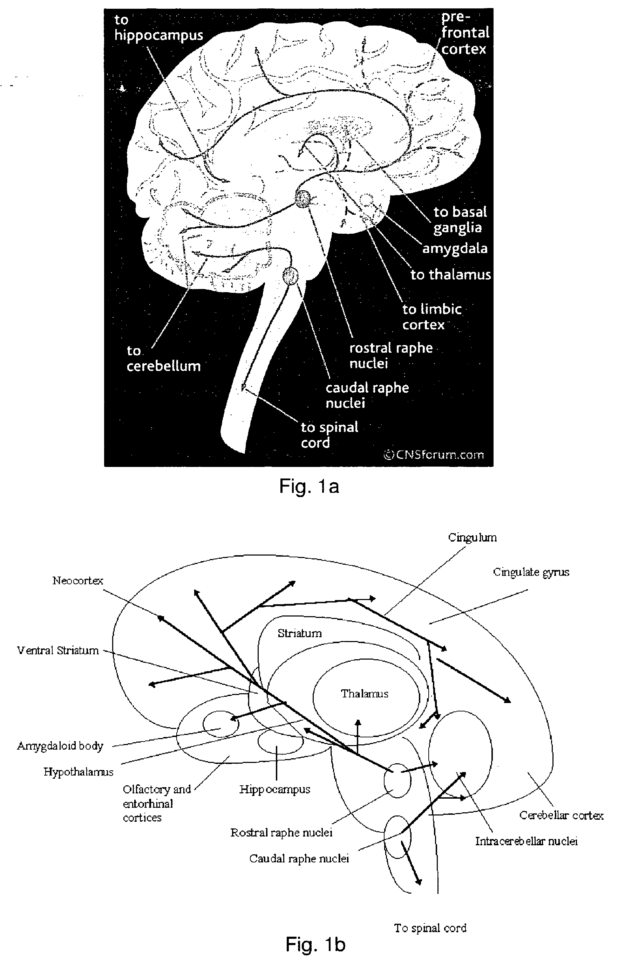

Fig. 1 a is a cross-sectional side view illustration of the human brain. The neuro-anatomical structures caudal raphe nuclei and rostral raphe nuclei are both part of raphe nuclei located in the brain stem. Raphe nuclei is a source of serotonin production in the brain and the arrow shows the natural distribution pathways of serotonin produced in caudal raphe nuclei and rostral raphe nuclei, respectively.

Fig. 1b is a cross-sectional schematic diagram of the human brain schematically illustrating specific neuro-anatomical regions and the serotonin pathways originating from raphe nuclei. The orientation of the brain in fig. 1b is reversed around a vertical axis compared to fig. 1a, e.g. cerebellum is in the left side of fig. 1a and in the right side of fig. 1b.

Fig. 4 is a cross-sectional side view illustration of the human brain. Substantia nigra and VTA are sources of dopamine production in the brain and the arrows show the natural distribution pathways of dopamine produced in substantia nigra and VTA, respectively. It is seen from the figure that substantia nigra provides dopamine to the striatum whereas VTA provides dopamine to the hippocampus, nucleus accumbens and the frontal cortex.

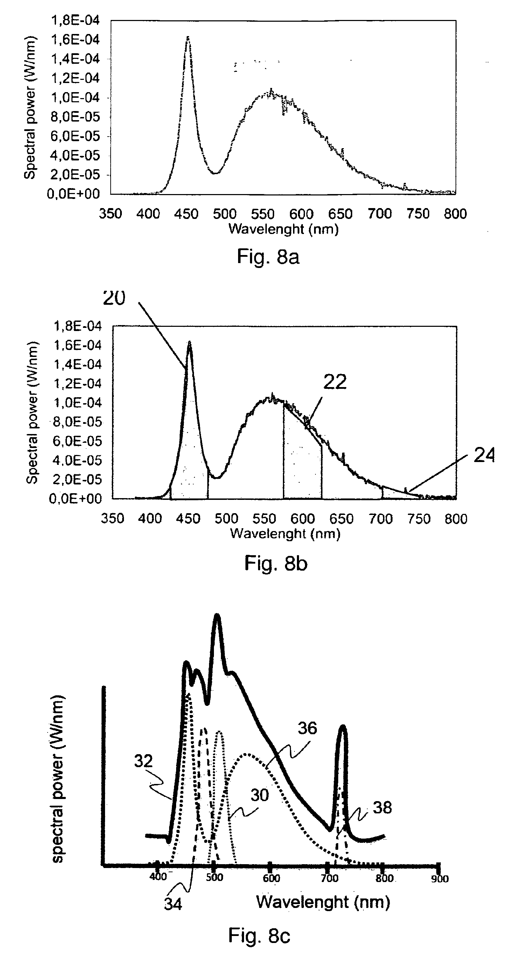

Figure 8a shows an example of the wavelength spectrum of a phosphor based white LED. These LEDs are provided by coating an LED of one color (mostly blue LED made of InGaN) with phosphor of different colors to form white light. Depending on the color of the original LED, phosphors of different colors can be employed. If several phosphor layers of distinct colors are applied, the emitted spectrum is broadened, effectively raising the color rendering index (CRI) value of a given LED. The X-axis of the graph in fig. 8a is the wavelength of the light in nanometers (nm) and the Y-axis is the spectral power in Watt / nm. In the graph the LED emits light with maximum power at two peaks of about 450nm and about 560nm.

Figure 8b shows example of how to calculate the spectral output emitted between certain wavelengths. The underlying spectrum is the same as in fig. 8a. Integrating from 425nm to 475nm results in a total power of about P₄₂₅₋₄₇₅ = 3.9 mW (the area indicated with 20 in the figure), integrating from 570 to 62 0nm results in a total power of about P₅₇₀₋₆₂₀ = 4.5 mW (indicated with 22 in the figure), and integrating from 700nm to 750nm results in a total power of P₇₀₀₋₇₅₀ = 0.37 mW of power.

Based on clinical trials it appears that both relative proportion of total power and/or energy of two or more frequency areas are important on medical impact to patient. I.e. ratios such as P₄₂₅₋₄₇₅ / P₇₀₀₋₇₅₀ and/or P₇₀₀₋₇₅₀ / P₅₇₀₋₆₂₀ or applied energy ratio such as t₁ x P₇₀₀₋₇₅₀ / t₂ x P₅₇₀₋₆₂₀ where t₁ and t₂ correspond to the time each power in each wavelength interval has been applied. Wavelength intervals might also overlap for example P₄₀₀₋₅₀₀ / P₄₅₀₋₆₀₀.

Based on research conducted by the inventors different neuro-anatomical regions of the brain have different presence, concentration and/or amount of light sensitive opsins (opsin 1, 2, 3 etc.). Each opsin typically has a characteristic absorption spectrum, i.e. most of the absorption of light happens in certain wavelengths as shown in Figure 2. The nature (e.g. the spectral composition) of the optical radiation therefore influences which type of substance(s) that is produced (e.g neuro transmitters, hormones etc.) or which chemical balance that is triggered. One embodiment of the invention therefore relates to the balance / ratio between energies in different wavelength intervals and the different medical impacts that results from this. Additionally there might be neural synapses with no opsins, which are activated by light.

Further, some opsins, such as neuropsin OPN5, might have bistable forms and therefore two or more absorption peaks. Furthermore, changes in gene reading can produce variants of the same protein; being close enough and functionally adequate, this mechanism may also produce opsin-variants of the same gene with other absorption peaks.

The basic process can then essentially be that a) light of a certain wavelength, b) creates a response in the brain, c) this will change neural transmission characteristics, d) this can lead to production of for example hormones or neural transmitters, e) said hormones or neural transmitters affect patients.

for example if the ratio of spectral output from different wavelength intervals tends towards shorter wavelengths, the production of a first neuro-transmitter / hormone is lower than the production of a second neuro-transmitter / hormone. On the other hand: if relative amount of energy coming from longer wavelength is higher than the amount of energy coming from shorter wavelengths, the relative production of the second neuro-transmitter / hormone might be lower than relative production of the first neuro-transmitter / hormone, i.e. the resulting chemical balance is influenced by the nature of the light.

The present invention is not limited to the use of two or more LEDs. The radiation units of a light therapy device according to the invention can be provided with a single LED with known fixed spectrum where this fixed spectrum is known to affect specific symptoms of one or more disorders. For example the spectrum of a "Blue color white LED" has a different spectrum than a "Red color white LED".

An example of creating different spectral power functions using several LEDs is shown in Figure 8c. In the example there are four LED's 30, 34, 36, 38, each with a characteristic wavelength spectrum. 36 is a white LED, 34 has a main peak at 490 nm, 30 has a main peak at 520 nm, 38 has a main peak at 730 nm. In case all LED's are ON at the same time a possible effective output spectrum 32 is created (the graph has been offset in the figure for clarity purposes).

If the power supplied to each of the four LEDs can be controlled independently, the power and energy of the peaks in the output spectrum 32 can be adjusted. As an example if 38 is shut down the amount of energy and power in near infrared area will be much lower than in case of having it on.

In general terms the device according to the present invention could have an arbitrary amount of light sources, such as LED's, each emitting light with a characteristic and known spectrum. Modification of the output power and the time the power is applied can alter the combined spectral output and thereby the effect on light therapy treatments can be altered.

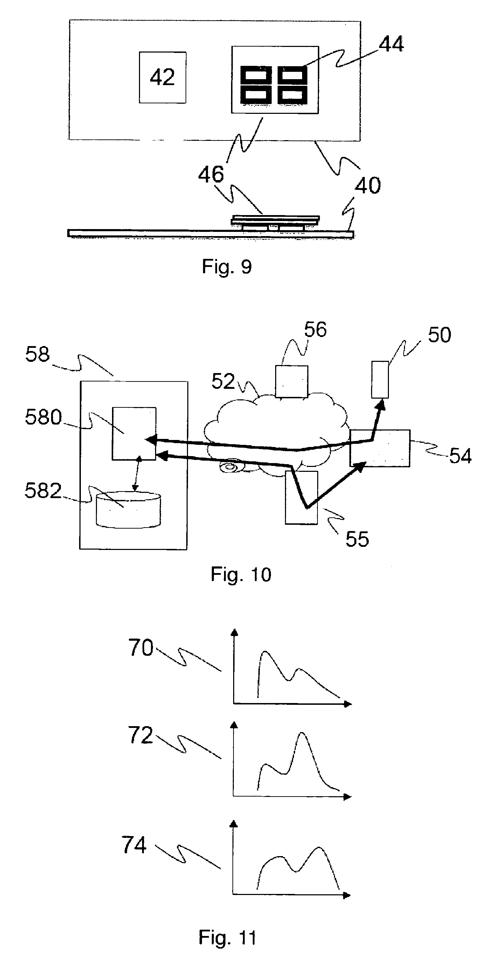

Figure 9 shows an example of a (part) of a radiation unit with four LED's (Light Emitting Diodes) 44 installed in a printed circuit board 40. The LED's can have also one or more phosphorous layers 46 on top of them. LED output power and timing can be controlled for example with a Digital Signal Processor (DSP) 42.

The light emitting device according to the invention can also have other form factors, such a lamp to be placed in the table or on the ceiling.

In order to control which applied energies in different wavelength intervals the device according to the invention can have a user interface for adjusting output power / energy levels / ratios in certain wavelength intervals. The light emitting device might further have a user interface to select which medical condition the light therapeutic treatment should apply.

The light emitting device might further have an interface to connect to an external device such as laptop to make modifications to the energy levels, wavelength intervals and/or ratio of spectral outputs. The device might further have an interface to connect to the Internet (directly or via PC (Personal Computer) / laptop or smart phone) to download new programs / energy level form factors or light therapy programs.

Figure 10 shows a high level architecture. The light therapy device 50 according to one embodiment of the invention can be connected via a personal computer 54 to server system 58 via Internet 52. The device 50 can be connected to PC 54 wirelessly using for example Bluetooth™ or WLAN (Wi-Fi) connectivity. The device 50 can be connected to pc 54 with wire such as Universal Serial Bus (USB) connectivity. The device 50 can be also connected directly to server system 58 via Internet 52 in case the device has connection means such as WLAN (Wireless Local Area Network) or for example integrated cellular modem. The device can be also be connected to Internet for example via Smart Phone or mobile phone. The device can be part of a mobile phone / smart phone or it can be accessory to a mobile phone / smart phone.

The server system 58 contains set of servers 582 either located centrally or distributed in as cloud service. Server can be for example run on Apache^{™}. Server system 58 can include database 584 such as MySQL or other relational or non-relational database. The purpose of the server system is to store settings related to light therapy devices 50. The server system can also host patient related data such as gender, age, symptoms, applied light therapies, results of the therapies. The system can host plurality of different type of light therapy programs for varying applied energy and timing for each feasible light wavelength range.

Based on embodiments of the invention there can be at least following high level type of light therapy programs
1) General programs which are feasible for most of the patient. These programs are typically programmed by doctors or other medical persons.
2) Customized programs based on patient responses to treatments. These programs can be programmed also by medical persons, but there might be feedback loop and possibility for patients to modify these programs. Programs can be programmed by patients using web interface and laptop 54. Persons can be for example provide information of used medicines and other illnesses.

The web interface is useful also in collecting possible side effect information.
3) Light therapy programs created / modified by users of and shared to other users. The sharing can take place by users uploading their experienced best practices to server system 58 via PC 54 or 56 over web interface. In addition users of personal computers (or smart phones etc.) can share in some embodiments the programs and feedback on programs via 3^{rd} party service 55 such as Facebook™ or other social networking platform.

Fig. 11 shows three different wavelength spectrums 70, 72, 74 of optical radiation, each having two intensity peaks but with different ratios of spectral outputs integrated around each peak. Each spectrum may have a different effect when applied trans-cranially into the brain. The three spectrums can e.g. be provided by a multi LED device.

### Example 1

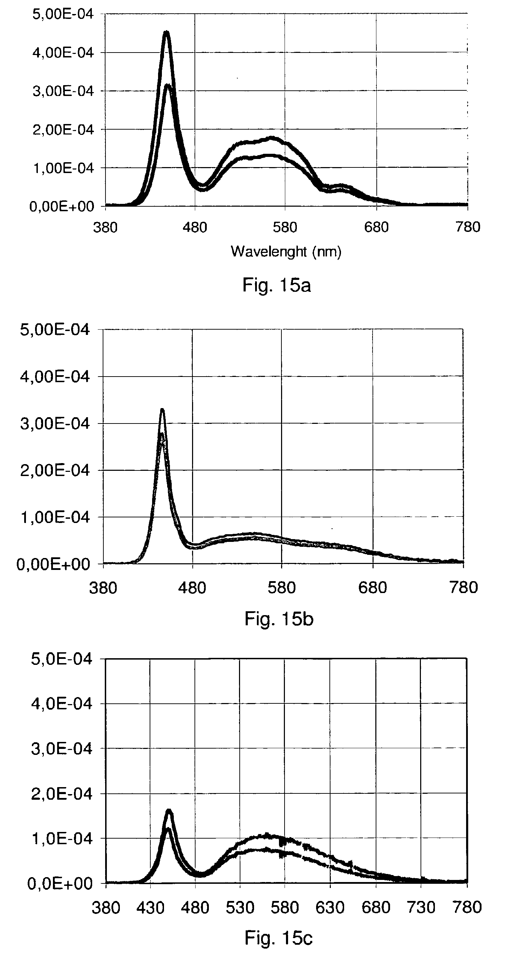

As an example the amount and ratio of blue and red light might be an important factor for treatment of SAD, anxiety, migraine and/or urinary incontinence. Figs. 15A-15E show various spectral compositions of LED's of certain types that has been tested for treatment puposes.

Figure 15A shows examples of measurement of the spectral power (Watt / nanometer) of two type "A" LED's used to treat patients. Type A spectra have been found in a number of conducted experiments to be efficient on curing SAD related symptoms. From the graph it can be seen that the blue peak wavelength is at approx. 450 nm with a half peak width of about 30 nm. The peak starts from about 410 nm and reaches to about 480nm. The "red" peak maximum value is lower with the peak power wavelength at about 566nm. Peak starts from about 500nm and goes up to 630nm. There is small additional peak at 644 nm. Additionally it has been found out that a LED spectrum resembling the spectrum in fig. 15A has been efficient on curing urinary incontinence. In some experiments type "A" has been found to cause side effects such as headache or migraine. The spectral output from 380 nm to 480 nm (blue peak) is 13.2 mW and 9 mW, respectively, for the two spectra (found by integrating the spectral power from 380 to 480 nm). The spectral output from 481 to 780 nm (red peak) is 21.2 and 16.1 mW, respectively, for the two spectra (found by integrating the spectral power from 481 to 780 nm). The total power is thus 34.4 mW and 25.1 mW, respectively. The ratio between the blue and red light of the two LEDs is thus 13.2/21.2 = 0.62 and 9/16.1 = 0.56, respectively.

Figure 15B shows examples of measurements of the spectral power (Watt / nanometer) of five type "B" LED's used to treat patients. Type B spectra have been found in a number of conducted experiments to be efficient on curing SAD related symptoms. From the graph it can be seen that the blue peak wavelength is at approx. 448 nm with a half peak width of about 30 nm. The peak starts from about 418 nm and reaches to about 480nm. There is some "red" light with the peak at 562nm. Additionally it has been found that Type B spectra have been efficient on curing urinary incontinence. For some patient type B spectra have been found to help on, e.g. reduce symptoms of, anxiety and migraine. The spectral output from 380 nm to 480 nm (blue peak) is between 6.1-7.8 mW for the five spectra. The spectral output from 481 to 780 nm (red peak) is between 8.5 and 10.9 mW for the five spectra. The total power is thus between 14.6 and 18.8 mW. The ratio between the blue and red light of the five type B LEDs are between 0.678 and 0.719.

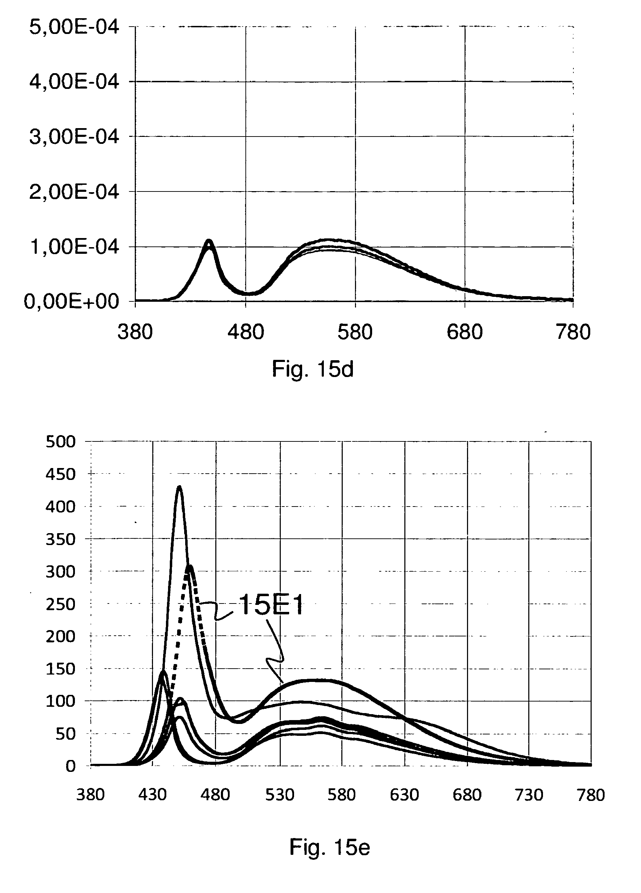

Figure 15ACshows examples of measurement of the spectral power (Watt / nanometer) of two type "C" LED's used to treat patients. Type C spectra have been found in a number of conducted experiments to be efficient on curing SAD related symptoms. From the graph it can be seen that the blue peak wavelength is at approx. 450 nm with a half peak width of about 30 nm. The peak starts from about 420 nm and reaches to about 480nm. There is a "red" light peak with the peak maximum at 562nm. Additionally it has been found that type C spectra have been efficient on curing urinary incontinence. Additionally it has been found in conducted experiments that type C spectra can treat symptoms of anxiety and migraine. The spectral output from 380 nm to 480 nm (blue peak) is 3 mW and 4.1 mW, respectively, for the two spectra. The spectral output from 481 to 780 nm (red peak) is 9.4 mW and 13.5 mW, respectively, for the two spectra. The total power is thus 12.4 mW and 17.6 mW, respectively. The ratio between the blue and red light of the two LEDs is thus 0.323 and 0.308, respectively.

Figure 15D shows examples of measurements of the spectral power (Watt / nanometer) of four type "D" LED's used to treat patients. Type D spectra have been found in a number of conducted experiments to be efficient on curing SAD related symptoms, however impact has not been as good as with LED types "A", "B" or "C". In addition type "D" has been found to cause side effect such as headache. From the graph it can be seen that the blue peak wavelength is at approx. 450 nm with a half peak width of about 30 nm. The peak starts from about 420 nm and reaches to about 480nm. There is a "red" light peak with the peak maximum at 562nm. The spectral output from 380 nm to 480 nm (blue peak) is between 2.5-3.2 mW for the five spectra. The spectral output from 481 to 780 nm (red peak) is between 12.2 and 15.2 mW for the five spectra. The total power is thus between 14.7 and 18.3 mW.

The ratio between the blue and red light of the five type B LEDs are between 0.208 and 0.22.

Figure 15E shows examples of measurements of the spectral power (Watt / nanometer) of a set of type "E" LED's used to treat patients. Type E spectra have been found in a number of conducted experiments to be efficient on curing SAD related symptoms. A spectrum 15E1 (dashed line, marked in figure) has been found to have an effect on anxiety and migraine. The ratio between blue peak integral to red peak integral for 15E1 is 0.56. Additionally it has been found that variation in manufacturing technologies used to produce LED's can lead to variation in the wavelength location of the maximum of the blue peak between 430-480 nm.

Additionally some patients have been exposed to "pure" blue, i.e. a spectrum having little or no red peaks (ratio >>1). Said spectrum has been found to cause side effects such as migraine to patients.

### Summary of studies

### Treatment of SAD and/or urinary incontinence

- The treatment should be conducted with LED spectrum which is at or below type "A" spectrum preferably close to type "B".
- The spectrum preferably has sufficient amount of "blue" color, i.e. higher than in type "D".
- In order to avoid non desired side effects the blue color peak maximum should be higher than red color peak maximum.
- Additionally it has been found that when the total power is too high or when red color peak power is too high there might be side effects relating to headache or migraine.
- Preferably, in order to avoid side effects the ratio between blue and red peaks should be higher than 0.25.
- Additionally the total power should be under 34 mW to avoid side effects (for used treatment times, i.e. to derive total energy).
- Preferably the spectrum should not be mostly blue.

### Treatment of migraine and/or anxiety

- Spectra types "B", "C" and 15E1 have been found useful, however the spectrum should preferably be type "B" or below.
- Peak power of the red peak should preferably not significantly exceed 0.1 mW/nm since higher red powers have been found to cause migraine in some patients. Preferably the ratio between integrals of blue peak vs red peak should be between 0.3 and 0.9.
- The total power should not be too high (preferably below 34 mW per ear) since it has been found to cause migraine as side effect.

In typical treatments timing used has been varying between 6 - 12 minutes. Treatment has been applied to both ears at the same time resulting in a total maximum energy of (in case of type "A") 2 x 12 min x 60 sec/min x 34 mW = 50 Joules and with minimum energy of 2 x 6 min x 60-sec /-min x 15.7 mW =11 Joules.

### Example 2

A study is being conducted to measure the acute effect of trans-cranial illumination on mouse brains. The aim of the study is further to measure the neuro endocrinological responses in the mouse brains.

A plurality of mice is randomized into two groups; one group is receiving trans-cranial light therapy via the auditory canals for eight minutes by means of device according to the present invention. A second group is wearing the light therapy device, however without receiving illumination from the device.

All mice are executed by cervical dislocation immediately after light therapy. Subsequently blood samples from cranial circulation and tissue samples from various neuro anatomical brain structures, PVA, Mesencephalon, Cerebral cortex, Medulla oblongata and Hippocampus are collected.

By analyzing the blood and tissue samples the amount of a number of hormones and neurotransmitters, such as dopamine, in the various neuro-anatomical brain structures can be determined. And by comparing the results from the two groups of mice, and testing for statistical significance, the acute effect of trans-cranial light therapy in mice can be determined, i.e. it can be directly confirmed that trans-cranial light therapy directed non-invasively from an extra-cranial position of a test subject results in altering of the amount, level, production, release, re-uptake and/or metabolism of dopamine in the brain of the test subject.

### Example 3

The patient (male, 70 years old) had been diagnosed with Parkinson's disease. Light therapy was applied to the patient using the device according to the invention. The light spectrum 15E1 as shown in the Figure 15E1 was applied through the ears of the patient for 12 minutes daily. During the treatment of two weeks the patient reported significant improvement, i.e. a significant reduction in symptoms related to Parkinson's. In the first phase (during first days of use) anxiety related to Parkinson's remitted. During the following weeks motor performance and cognitive speed was observed to improve and motor performance (tremor was decreased) and overall movement speed increased. Also the observer (the patient's adult son) noticed improved speech rate, increased speed of thinking and improved mood.

The patient himself reported immediate anxiolytic effect of the light therapy, and experienced that eased anxiety was related to Parkinson's also. The patient reported overall increased quality of life and was able to perform daily tasks on days when light was administered. Overall the light treatment helped, cured and reduced symptoms related to Parkinson's and since Parkinsons's is intimately related to lack of dopamine (as stated previously herein) it may be concluded that the applied light therapy had an effect on dopamine production in the brain. Thus, with an exemplary embodiment of the device according to the invention, light therapy was directed non-invasively at substantia nigra of a patient from an extra-cranial position, i.e. through the ear-canal, which resulted in altering of the production, release, re-uptake and/or metabolism of dopamine in substantia nigra of the user.

### Example 4

A study was conducted where over a four week period with a daily 8-12 minute dose, 92% of the patients with SAD achieved full remission measured by the self-rated BDI-21, the most widely used questionnaire for rating depression. The daily time of usage was personalized at the study clinic.

### Example 5

Trans-cranial light therapy was applied to three male patients, each patient approx. 40 years old.

The first patient was consuming alcohol in high amounts daily; this patient was possibly suffering from alcoholism. Light therapy was applied trans-cranially once per day (12 min daily dose) via the ear-canal using a device according to the invention. The applied light spectrum corresponds to the spectrum illustrated in fig. 15C. After being treated with the light therapy he reported to have lost the desire to consume alcohol or alcoholic beverages. Even after the light therapy treatment stopped the alcoholic intake was drastically reduced compared to the initial abusive amounts.

The **second** patient, a highly trained generally healthy medical professional, performed a series of tests on himself: he consumed alcohol in small amounts (on the order of one standard drink) and rated the pleasure he felt before and after alcohol intake. This was on some days combined with a daily dose of light therapy via the ear canal using the light spectrum 15E1 as illustrated in fig. 15E. He reported a significant decrease in subjectively felt pleasure of alcohol intake on days when light therapy was applied.

The **third** patient was generally in a healthy condition. He took several daily doses (1-3 daily doses, 12 minutes each) applying the light spectrum 15E1 as shown in fig. 15E using a device according the invention. The patient experienced a total lack of desire to smoke. Furthermore, these high daily amounts of trans-cranially applied light caused a status, where the psychological reward mechanisms were reduced, such as enjoyment of working and sexual desire / libido. Reduced libido has also been experienced by other patients using several 12 min doses of light therapy daily.

Obsessive compulsive disorders (OCD), alcohol addiction and nicotine addiction are all related to the dopamine system. As trans-cranial applied light therapy provides significant effects on these disorders/addictions it can be concluded that the light therapy has an effect on the dopamine production.

### Example 6

In order to study the impact of light therapy to the cognitive performance of humans a commonly used neuropsychological test called "trail-making test" was conducted with eleven test persons being subject to light therapy treatment.

A trail-making test is a neuropsychological test of visual attention and task switching. The task requires the test person to 'connect-the-dots' of a plurality (e.g. 25) consecutive targets on a sheet of paper or computer screen. Two versions are available: A, in which the targets are all numbers (1, 2, 3, etc.), and B, in which the subject alternates between numbers and letters (1, A, 2, B, etc.). The goal of the test person is to finish the test as quickly as possible, and the time taken to complete the test is used as the primary performance metric. The trail making test has become a common diagnostic tool in clinical settings.

In this example eleven test persons in normal healthy condition were subject to transcranial applied light therapy using a device according to the invention. The light spectrum shown in figure 15A was applied for a period of four weeks with a daily dose of 12 minutes for 5 days a week (not weekends). Trail-making tests of types A and B were conducted before and after four the week treatment period.

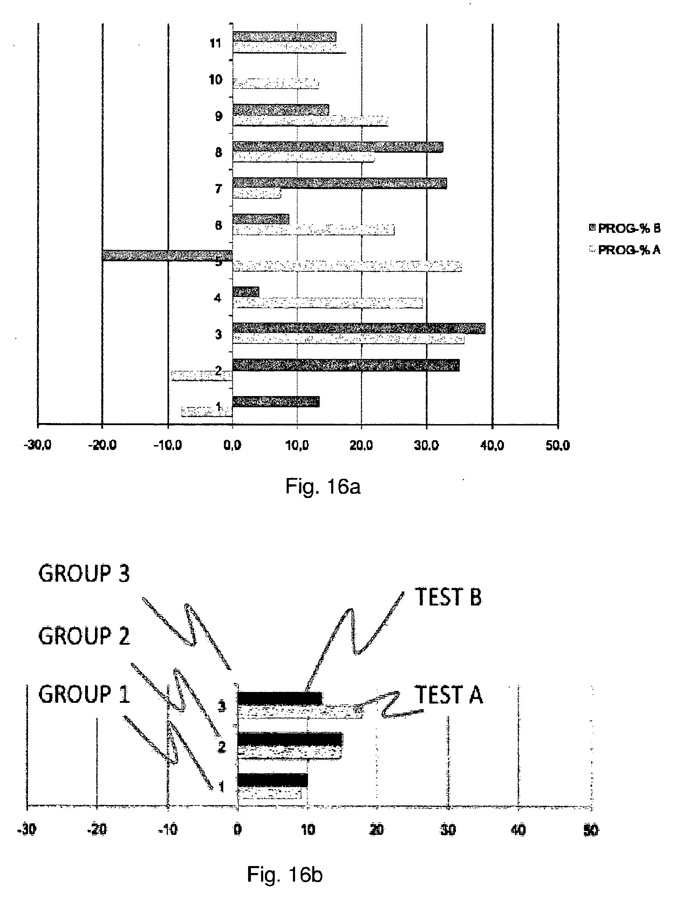

Results are illustrated in fig. 16A. The eleven test persons along the vertical scale and with the measured progress for type A and type B trail making test. I.e. test person no. 11 performed approx. 16 and 17 percent better on the type B and type A trail making test, respectively, after the four weeks of light therapy, whereas test person no. improved his type A test with 35 percent but he conducted the type B test 20 percent slower after light therapy treatment. Overall 9 test persons out of 11 performed clearly better on type A test after the light therapy treatment. Two of the test persons showed lower performance on type A. 9 out of 11 performed clearly better on B type test after the treatment period. One performed worse than average. One did not show change in performance.

Dopamine is known to be related to the cognitive performance. Thus, since light therapy had a clear impact on cognitive performance of the healthy test persons it can be concluded that light therapy affects dopamine production in the brain.

### Example 7

As a continuation of the previous example 5 a group of 90 generally healthy test persons were subject to trans-cranial applied light therapy using a device according to the invention. The light spectrum shown in figure 15D was applied for a period of four weeks with a daily dose of 12 minutes for 5 days a week (not weekends). Trail-making tests of types A and B were conducted before and after four the week treatment period. The test persons were divided into three groups with 30 persons each. A different light intensity of the light was applied to each group of test persons during the four weeks. Group 1: one lumen, group 2: four lumens and group 3: nine-lumens of light. Results are illustrated in fig. 16B. Overall the three groups performed 10-15 percent better in both tests compared to their performance before the light therapy. There are no significant differences between the groups, however with a small trend towards larger improvement with higher intensity of the light.

Dopamine is known to be related to the cognitive performance. Thus, since light therapy had a clear impact on cognitive performance of the healthy test persons it can be concluded that light therapy affects dopamine production in the brain.

### Example 8

A double blind, placebo controlled, test for motoric reaction time was conducted with two groups of 11 persons (a total of 22). The test persons were male 18-32 years top athletes (national ice hockey league players). The test persons were subject to transcranial applied light therapy using a device according to the invention. The light spectrum in figure 15e with the highest relative peak power was applied for a period of four weeks with a daily dose of 12 minutes for 7 days a week.

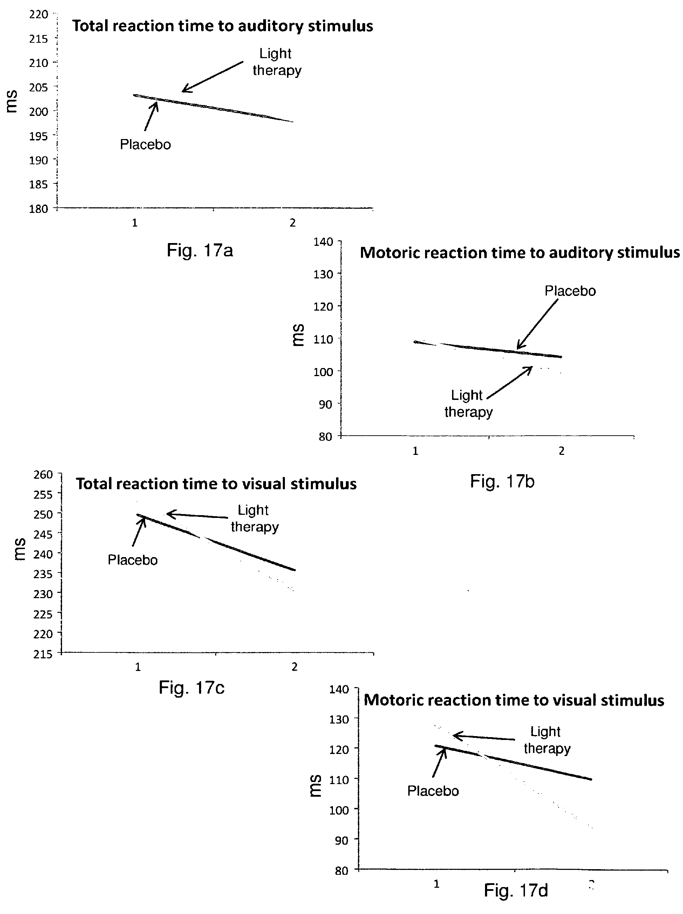

Generally ice hockey players are athletes with extremely low reaction times due to the nature of the sport. In spite of this the light therapy improved the reaction times of these very fast athletes. The results of the tests are illustrated in figs. 17a-d with total reaction time to auditory stimulus in fig. 17a, motoric reaction time to auditory stimulus in fig. 17b, total reaction time to visual stimulus in fig. 17c and motoric reaction time to visual stimulus in fig. 17d. The mark "1" on the x-axis marks the average result of the test conducted prior to any light therapy whereas "2" marks the result of the test conducted after the period of light therapy. From the figures it can be seen that both the placebo group and the light therapy group show improvement in the measured reaction times. However, the improvement of the light therapy group is significantly better.

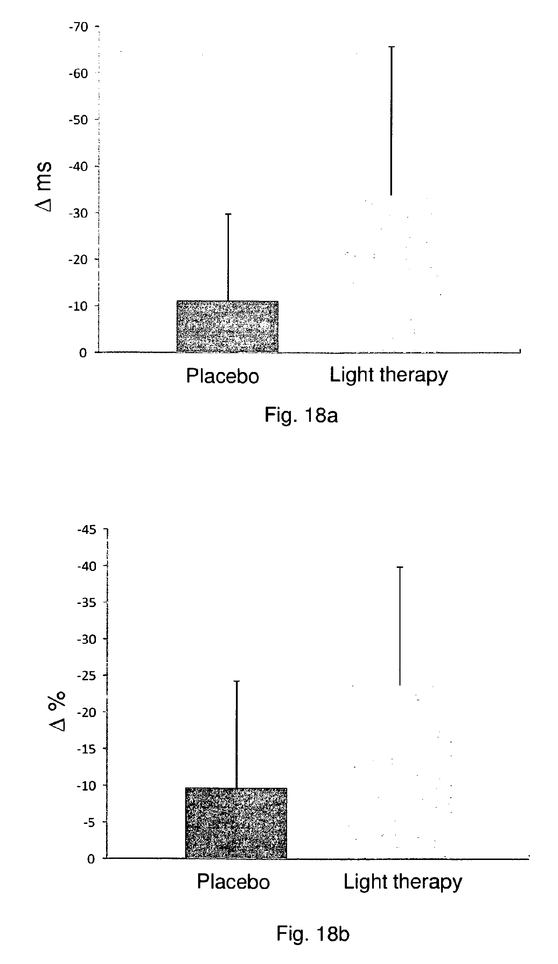

The improvement for motoric reaction time to visual stimulus is illustrates in fig. 18 with the absolute improvement in ms (milliseconds) in fig. 18a and the relative improvement in fig. 18b. From fig. 18b it is seen that the placebo group showed a 10% improvement whereas the group the underwent trans-cranial light therapy showed an improvement of almost 25%. Thus a significant statistical improvement in the motoric reaction time with a difference between placebo and the light therapy group of p=0.023, when adjusted for age and p=0.044 without adjustment. The difference in enhanced reaction time was almost three fold.

Dopamine is known to be related to the motoric performance and reaction time. Thus, since light therapy had a clear impact on motoric performance and reaction time of the healthy test persons it can be concluded that light therapy affects dopamine production in the brain.

### Example 9

A controlled dose-response study on the putative effect of extra visual bright light in the reduction of depressive symptoms in patients suffering from winter SAD was conducted. The bright light was administered trans-cranially via the ear canal daily during a four-week trial. Data was gathered during the darkest period of the year in northern Finland (latitude 64°01'N) where the amount of daily light is very short.

The seasonal pattern of recurrent episodes of depression has become known as Seasonal Affective Disorder (SAD). The precise pathogenesis of SAD is still uncertain, despite several explanatory theories such as photoperiod and phase-shifted circadian rhythms, neurotransmitter functions, and genetic hypothesis. Given the fact that winter SAD is far more prevalent than summer SAD, the term SAD usually refers to winter SAD and is used accordingly hereafter. Episodes of SAD peak in winter and are characterized by typical and atypical depressive symptoms, i.e. lowered mood, energy loss, excessive sleep with difficulty waking, craving for carbohydrates, weight gain, irritability, social withdrawal, daytime fatigue, and loss of concentration.

The prevalence of SAD varies from 0 to approx. 10% in the general population. Climatological, social and cultural influences, genetic factors and geographical latitude have been reported to have an impact on the prevalence of SAD. SAD is more common among females and younger adults. SAD in females is usually characterized by minor depressive episodes, whereas males more commonly experience major depression.

Many controlled studies have found bright light therapy (BLT) effective in treating SAD [46;9;33]. In a systematic review and meta-analysis, the effect size for the reduction of depressive symptoms by BLT in the treatment of SAD was 0.84 [12]. The antidepressant effect of bright light is potentiated by early morning administration in circadian time, about 2.5 hours after the sleep midpoint [42]. According to the clinical guidelines, the recommend bright light exposure in treatment of SAD is 10,000 lux for 30 min per day [20]. Although BLT is effective, about 70% of SAD patients complain that sitting in front of the bright light is uncomfortable [29], and almost one in five SAD patients stop BLT because of that [37].

The mechanism of action of BLT in the treatment of SAD is still under debate [7,2], and it is widely believed that the effect of light is mediated via the eye [50]. However, there is evidence that in mammals significant amounts of light penetrate the skull bone and reach the brain [49].

### Subjects and methods

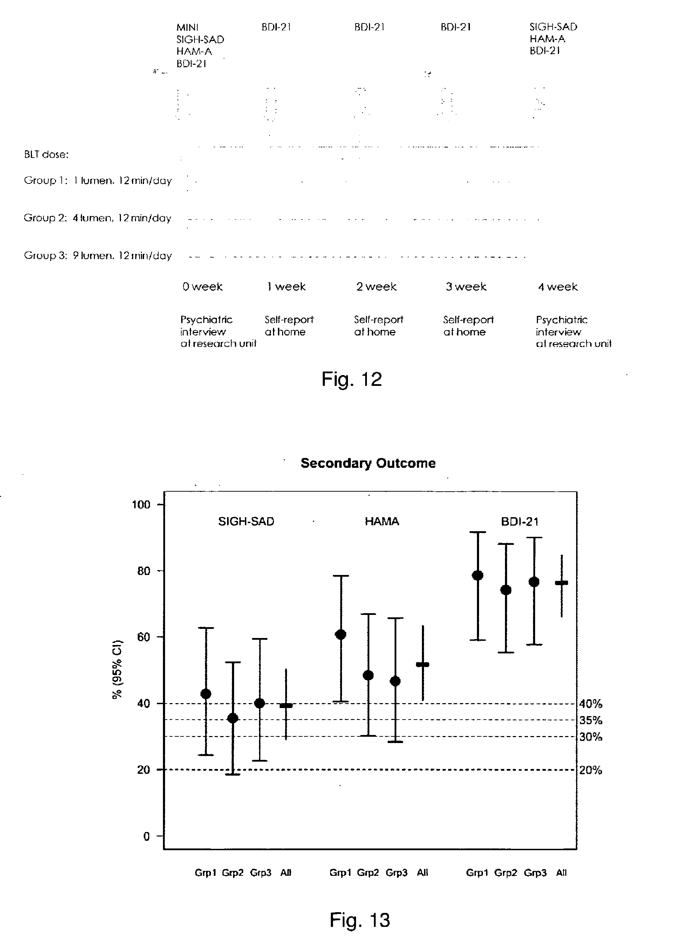

Adult persons suffering from seasonal depressive symptoms were recruited through advertisements in a local newspaper and were pre-screened for symptoms of SAD by a phone interview. The procedure of the study is presented in fig. 12.

Structured diagnostic interviews were conducted at week 0 and 4 by two trained psychiatrists. Diagnosis according to the Diagnostic and Statistical Manual of Mental Disorders (DSM-IV) [1] for recurrent major depression (moderate or severe) was obtained using the Mini International Neuropsychiatric Interview (MINI) [38]. In addition, patients had to fulfil the diagnostic criteria for "seasonal pattern" [1].

The subjects included in the study had to score at least 20 points on the 29-item Structured Interview Guide for the Hamilton Depression Rating Scale - Seasonal Affective Disorder (SIGH-SAD) [51], with a 21-item Hamilton Depression Rating Scale (HAMD-21) score of 10 or more and eight-item atypical symptom score of 5 or more. In this study, inclusion scores were analogous with the criteria used for evaluating the response to treatment in patients with SAD in earlier studies [48,46]. Subjects with lifetime psychotic disorders, bipolar disorders, severe personality disorders, substance abuse or dependence, suicidal ideation during the past month, any psychotropic medications, and other bright-light therapy for the current SAD episode were excluded from this study. Pregnant females were also excluded. Written informed consent was obtained from the subjects after they had been given a full description of the study at the first visit during week 0. The research protocol was approved by the Ethics Committee of Oulu University Hospital, Finland.

### The light therapy device

The brain-targeted bright light treatment was given transcranially via ear canals by using the Valkee brain stimulation headset. This device was approved as a medical device in the European Union on 30 March 2010, and since then it has been available for customers in Finland and other EU countries. The light was produced using light-emitting diodes (white LEDs), which were attached to earplugs. The bright light was transmitted to the ear canal by an optical guide. Daily BLT (bright light therapy) was taken during the forenoon at home, and each treatment session lasted 12 minutes.

### Grouping of subjects

The subjects involved in the study were randomly divided into three groups: low dose (group 1), intermediate dose (group 2) and high dose BLT (group 3). The randomization procedure followed a double-blind design. The amount of received light in the three groups was 1 lumen, 4 lumen and 9 lumen, respectively. Lumen is a measure of luminous flux, which is defined as the total amount of visible light emitted from a light source through a solid angle.

### Measurement of SAD

The sum score of SIGH-SAD was used to evaluate the severity of SAD. The remission criterion was defined as score of ≤ 8 of 29-item SIGH-SAD score at week 4. For further analysis, SIGH-SAD, 14-item Structured Interview Guide for the Hamilton Anxiety Rating Scale (HAMA) [14] and 21-item Becks Depression inventory, BDI [3] where used to evaluate the response to treatment. The criterion for response was fulfilled when the patient had a decrease of 50% or more from the baseline scores in SIGH-SAD, HAMA and BDI. In addition to measuring safety and tolerability, information of bright light-related adverse events was gathered.

### Statistical analyses

All data are presented as percentages or as mean with 95% confidence intervals. Student t-test was used to compare baseline values between genders within light treatment groups. Categorical variables were compared by Chi-Square test or Fisher's Exact test when appropriate. The within-group and between group changes in variables during the study were analysed with repeated measures analysis of variance (ANOVA). Results with 2-sided p values <0.05 were considered statistically significant. Statistical analyses were performed using SAS version 9.2.

### Results

Ninety patients with SAD, 68 of whom were females, participated in this study. Of these, one female patient dropped out due to a trip abroad. The mean age of participants was 43.0 years (SD 10.9, range: from 22 to 65 years). Table 1 shows the demographic and baseline variables of the treatment groups in both genders. The groups were similar in most respects, but differed in some variables. Statistically significant differences were found in age and BDI baseline sum score in treatment group 3 and in SIGH-SAD baseline sum score in treatment group 1 between females and males.

**Table 1: Demographic and baseline variables for treatment groups**

| Variables | Treatment group | Female (N) | | Male (N) | | p-value |
|---|---|---|---|---|---|---|
| | | Mean | SD | Mean | SD | |
| Age | 1 | 42.0 (22) | 10.6 | 43.3 (6) | 10.8 | 0.7952 |
| | 2 | 42.9 (25) | 10.1 | 49.3 (6) | 11.3 | 0.1795 |
| | 3 | 38.4 (20) | 10.2 | 51.0(10) | 11.3 | 0.0045 |
| SIGH-SAD | 1 | 37.8 | 5.5 | 32.0 | 7.2 | 0.0414 |
| | 2 | 36.9 | 6.3 | 32.5 | 6.8 | 0.1407 |
| | 3 | 36.3 | 6.1 | 34.4 | 8.8 | 0.4092 |
| HAM-A | 1 | 24.0 | 6.1 | 22.2 | 5.7 | 0.5034 |
| | 2 | 23.0 | 6.5 | 21.0 | 7.7 | 0.5182 |
| | 3 | 22.2 | 5.7 | 20.9 | 6.3 | 0.8619 |
| BDI | 1 | 20.5 | 8.3 | 17.5 | 8.0 | 0.2784 |
| | 2 | 19.7 | 8.1 | 15.7 | 10.4 | 0.3057 |
| | 3 | 22.2 | 8.7 | 13.7 | 8.7 | 0.0185 |

When compared to the baseline (week 0), statistically significant decreases were found in mean SIGH-SAD total scores after adjusting for age and gender in each treatment group (Table 2). The mean SIGH-SAD total scores decreased 17.6 points (47.4%, p<.0001), 17.0 points (45.9%, p<.0001) and 15.9 points (43.7%, p<.0001) in the three treatment groups (1, 4, 9 lumen), respectively. The corresponding values for HAMA were 12.0 (49.9%, p<.0137), 11.4 (49.5%, p<.0056), 10.1 (46.5%, p<.0001) and for BDI 13.7 (67.3%, p<.0158), 13.4 (67.4%, p<.1282), 11.9 (63.2%, p<.0013). Although subjects in each group improved after exposure to bright light treatment, there were no statistical differences between these improvements.

**Table 2: Depression and anxiety scale measures over four week study period**

| Measure | Group 1 (N=28) | | Group 2 (N=31) | | Group 3 (N=30) | |
|---|---|---|---|---|---|---|
| | Mean | 95%Cl | Mean | SD | Mean | SD |
| Structured Interview Guide for the Hamilton Depression Rating Scale | | | | | | |
| Seasonal Affective Disorder Version (SIGH-SAD) score | | | | | | |
| Baseline | 36.6 | 34.1-39.0 | 36.1 | 33.7-38.5 | 35.7 | 33.5-37.8 |
| Endpoint | 19.0 | 14.4-23.6 | 19.1 | 15.5-22.7 | 19.8 | 15.6-23.9 |
| | | | | | | |
| Hamilton Depression Rating Scale (HAMD) score | | | | | | |
| Baseline | 21.8 | 20.2-23.5 | 21.5 | 19.5-23.5 | 21.1 | 19.6-22.6 |
| Endpoint | 11.3 | 8.5-14.1 | 11.1 | 9.0-13.1 | 11.5 | 8.9-14.0 |
| | | | | | | |
| Atypical Symptom Scale score | | | | | | |
| Baseline | 14.8 | 13.1-16.4 | 14.5 | 13.2-15.9 | 14.6 | 13.2-16.0 |
| Endpoint | 7.7 | 5.3-10.1 | 8.0 | 6.2-9.9 | 8.3 | 6.3-10.3 |
| | | | | | | |
| Hamilton Anxiety Rating (HAMA) Scale | | | | | | |
| Baseline | 23.6 | 21.3-26.0 | 22.6 | 20.2-25.1 | 22.1 | 19.9-24.2 |
| Endpoint | 11.6 | 8.2-14.9 | 11.2 | 8.7-13.7 | 12.0 | 9.1-14.8 |
| | | | | | | |
| Beck's Depression Inventory | | | | | | |
| Baseline | 20.6 | 17.5-23.7 | 18.9 | 15.8-22.1 | 19.3 | 15.8-22.9 |
| Endpoint | 6.9 | 3.6-10.1 | 5.5 | 3.2-7.9 | 7.4 | 4.4-10.4 |
| | | | | | | |
| | % | 95%Cl | % | 95%Cl | % | 95%Cl |
| SIGH-SAD improvement | 47.4 | 34.9-60.0 | 45.9 | 36.0-55.7 | 43.7 | 32.7-54.8 |
| HAMA improvement | 49.9 | 34.7-65.2 | 49.5 | 38.5-60.5 | 46.5 | 35.9-57.2 |
| BDI improvement | 67.3 | 53.0-81.6 | 67.4 | 55.5-79.4 | 63.2 | 49.9-76.6 |
| | Proportion | 95%Cl | Proportion | 95%Cl | Proportion | 95%Cl |
| SIGH-SAD score ≤8 | 28.6 | 10.7-46.6 | 16.1 | 2.4-29.8 | 13.3 | 0.4-26.2 |

The proportions of the patients in each group achieving 50% or greater improvement in SAD symptoms are shown in fig. 13. The response rate measured by SIGH-SAD varied from 35% to 45%. Corresponding variations for HAMA were 47-62% and for BDI 74-79%. Although the response rate was remarkable on each measurement, no statistically significant differences were found between treatment groups.

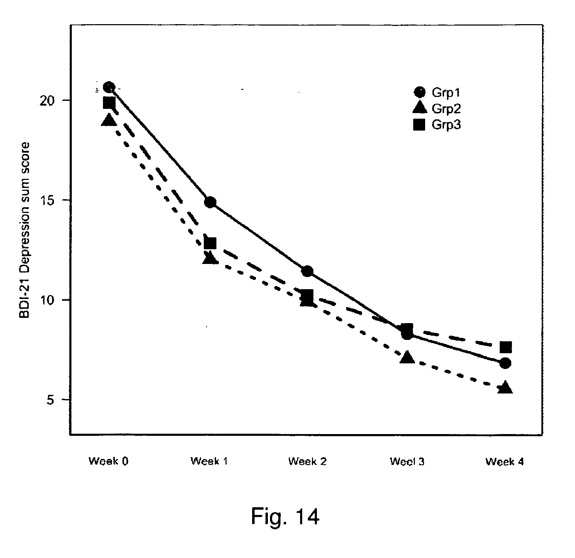

The self-rated BDI was assessed weekly in order to evaluate patients' depressive symptoms throughout the study (Fig. 14). A statistically significant decrease was found in each treatment group already at week 1 when compared to baseline, and the decrease continued throughout the study, although the decrease in depression scores did not differ between treatment groups.

The proportion of patients who reported potential bright light-related adverse events was 28.1 % (n= 25). There were no statistically significant differences in emergence of bright light-related adverse events between treatment groups. The most common adverse events were temporary headache, insomnia and nausea, which were reported by 10.1%, 5.6% and 3.4% of the patients, respectively. The other symptoms reported were dizziness, earache, abnormal sensation in the maxillary region, tinnitus, tiredness, irregular heartbeat and irritability.

### Discussion

In this study it was found that both self-rated and psychiatrist-rated depressive and anxiety symptoms of SAD patients decreased significantly during the 4-week study period even after controlling for age and gender. However, there were no significant differences in improvement of anxiety or depressive symptoms between groups receiving different intensity of bright light via ear canals.

Bright light therapy has been reported to reduce depression symptoms as measured by the rating scales used in this study [46]. The significant reduction in the symptoms of SAD in the present study parallels earlier two- to four-week studies with bright light therapy using traditional bright light devices. Comparing decreases of symptoms across studies is not optimal, but the comparison may be instructive. In the present study, when measured by SIGH-SAD-29, the decrease of raw scores varied between 15.9 and 17.6 points in three treatment groups, whereas in two earlier studies using same rating scale, the decrease was 10.4 points [22] and 15.1 points [46]. When comparing the percentage improvement, the percent change in the present study, ranging between 44% and 47%, is in between the improvements seen in two earlier studies, i.e. 57% [46] and over 30% [22]. The SIGH-SAD response-rates observed in

the present example are slightly lower than those seen in two earlier bright light studies using the same response criteria, i.e. 50% [2] and 63% [46].

Researchers have found that bright light exposure also has anxiolytic effects among clinically anxious adults and patients suffering from winter depression [52,43,9]. To the best of our knowledge, anxiety measurements have rarely been used in earlier bright light studies even though anxiety symptoms are quite common among patients suffering from depression. In the present study psychiatry-rated anxiety symptoms assessed by Hamilton anxiety scale (HAMA) decreased from moderate anxiety level to normal level [14] during the four-week study period in each treatment group. The decrease in anxiety symptoms is comparable to the earlier pharmaceutical studies in the treatment of generalized anxiety disorder (GAD) [40,18,15]. The decrease in the mean HAMA score in the present study ranged from 10.1 to 12.0 points in three treatment groups, whereas in earlier pharmaceutical studies using pregabalin (600mg/day) duloxetine (60-120mg/day) and venlaflaxine (75-225mg/day) the decrease of mean HAMA score was 11.6, 12.8 and 12.4 points, respectively [40,18,15]. The response rates in the present study varied from 47% to 62%, while ranging from 39% to 59% in earlier GAD studies using pregabalin [40,30,10,32], from 40% to 65% with duloxetine [36,18,15,28], and from 54% to 61 % with venlaflaxine [15,28]. In future studies it would be beneficial to utilize valid anxiety measurements as well when evaluating the effects of bright light in SAD.

When self-reported BDI was used, our findings were in line with bright light groups in earlier studies [11,26,9], showing a decrease of raw scores from moderate depression level to level of minimal depression symptoms [3]. The magnitude of the percentage improvement of depressive symptoms (from 63% to 68%) in the three treatment groups was comparable to the percent change in earlier bright light studies, i.e. 62% (BDI-25) [9], 65% (BDI-21) [11] and 69% (BDI-II) [26]. The BDI response rates in the present study are in between the response rates seen in earlier bright light studies, i.e. 58% [9] and 82% [26].

The proportion of the subjects who met the criterion of remission in the present study varied from 13% to 29% in the three treatment groups, whereas in earlier studies using the same remission criteria, the proportions were 47% [43], 42% [46] and 28% [2]. On the other hand, it is known that bright light treatment is less sufficient for more severely ill patients [37] and the severity of symptoms at baseline has effects on remission rates; SAD patients who experience more severe symptoms have lower remission rates [47]. In addition, a low atypical balance score may also be a poor prognostic sign for response to bright light therapy [44]. The SAD patients in the present study were more severely ill at baseline than reported in earlier studies. In the present study the baseline SIGH-SAD total scores varied from 35.7 to 36.5, whereas in three earlier SAD studies SIGH-SAD total scores ranged between 26.5 and 30.6 points [46,43,2]. In addition, in the present study, the possibility of spontaneous remissions was diminished by excluding patients diagnosed with bipolar disorder not otherwise specified or bipolar II disorder.

The adverse effects of bright light are generally known to be mild and rarely lead to treatment discontinuation [33]. However, 47% of SAD patients are refractory to light therapy [47], at least partly because of poor long-time compliance with light use [34]. Traditional light therapy requires a considerable daily time commitment from the patient during the symptomatic months, but it has been observed that 58% of patients stopped the light treatment when using the light device become voluntary [37]. In this study, transcranially administered BLT had an equal rate of potential adverse events (28%) when compared to earlier traditional BLT studies (25%) [45]. The most common side effects in this study were occasional headache, insomnia and nausea. In an earlier study using traditional BLT the emergence of headache was slightly lower, whereas insomnia and nausea were noted markedly more often [45]. We think that this new bright light innovation might result in better compliance than traditional BLT, because it is convenient, allows moving during treatment, does not irritate the eyes, and the daily treatment times are relatively short.

A limitation of this study is that we did not have a control group. We agree with Meesters and associates [26] that it is impossible to create real placebo condition for visible light. We are also aware of the fact that the bright light used in treatment of SAD is accompanied by a potentially large placebo response ranging from 21 % to 41 % [9,21,8]. It is generally assumed that sham devices have higher response rates than placebo pills used in trials of treatment of depression [17]. However, Brunoni and associates [5] reported in their large review and meta-analysis that repetitive transcranial magnetic stimulation (rTMS) as a non-pharmacological treatment in major depression had a lower placebo response than pharmacological therapy. The placebo-response of the device used in this study has not been explored so far. Since treatment sessions in tbrs study resemble the sessions of rTMS treatment, we believe that the improvement of depression and anxiety symptoms observed in our study are unlikely to be solely explained by the placebo effect. In future studies the size of the placebo response should be carefully scrutinized.

Some methodological limitations deserve discussion. There were older males than females and more severely ill females than males in one subgroup. In addition, the proportion of males (24.7%) was quite low. This may have biased our results, since it is known that the prevalence and severity of SAD may differ in different gender and age groups [37,4]. Moreover, it is found that males seem to underreport SAD symptoms [23].

We are aware that the amount of daylight increases towards the spring. However, our study was conducted during the darkest season of the year. All patients lived in Northern Finland, which is located only about 170 km south of the Arctic Circle. In addition, the majority patients were indoor workers who hardly saw daylight during the study period, which is strength of our study.

In sum, this is the first randomized controlled clinical trial to show antidepressant and anxiolytic effect of transcranial bright light therapy on symptomatic SAD patients. We are thus not able to compare our results to earlier studies of transcranial light in the treatment of SAD. These results are however in line with the findings of earlier bright light studies using traditional bright light devices in the treatment of SAD and pharmaceutical studies in the treatment of GAD. In future, studies on neuroimaging, neurobiology and placebo-controlled trials are called for to further assess the efficacy and mechanism of action of transcranially delivered bright light.

The conclusions of this randomized controlled trial where 89 subjects suffering from severe seasonal affective disorder had a 12-min daily Valkee dose at home in three different randomly divided groups of one, four, and nine lumen, are that the response rates in the sub-groups were 74-79% for seasonal depression and 47-62% for anxiety symptoms, and included at least 50% reduction in BDI-21 and HAMA score at week four. The daily administration time was fixed to the morning, after waking up.

## Claims

1. A device for non-invasive light therapy comprising one or more radiation units adapted to direct optical radiation at one or more neuroanatomical brain structures of a user through at least one auditory canal, **characterised in that** the device is adapted to emit the optical radiation from a radiation unit with a predefined ratio of a first spectral output power integrated over a first wavelength interval from 410 nm to 480 nm, and a second spectral output power integrated over a second wavelength interval from 485 nm to 700 nm, and wherein said ratio is between 0.45 and 0.65.

2. The device according to claim 1, further comprising a housing in optical and/or electrical connection with said one or more radiation units and one or more light sources for generating the optical radiation.

3. The device according to any of the preceding claims, further comprising means for adapting the intensity of the optical radiation.

4. The device according to any of the preceding claims, further comprising means for adapting the spectral composition of the optical radiation.

5. The device according to any of the preceding claims, wherein the optical radiation emitted from a radiation unit is applied with a predefined spectral composition.

6. The device according to any of the preceding claims, wherein the optical radiation emitted from a radiation unit is applied with one or more of the following parameters being predefined for a plurality of predefined wavelength intervals: duration, intensity, total power, spectral output and spectral composition.

7. The device according to any of the preceding claims, wherein said predefined ratio is between 0.45 and 0.5, or between 0.5 and 0.55, or between 0.55 and 0.6, or between 0.6 and 0.65.

8. The device according to any of the preceding claims, wherein said first wavelength interval has a start point of 410 nm, 415 nm, 420 nm, 425 nm, 430 nm or 435 nm, and an endpoint of between 450 nm, 455 nm, 460 nm, 465 nm, 470 nm, 475 nm or 480 nm.

9. The device according to any of the preceding claims, wherein said second wavelength interval has a start point of 485 nm, 490 nm, 500 nm, 505 nm, 510 nm, or 515 nm, and an endpoint of 600 nm, 610 nm, 620 nm, 630 nm, 640 nm, 650 nm, 660 nm, 670 nm, 680 nm, 690 nm or 700 nm.

10. The device according to any of the preceding claims, wherein the optical radiation is generated by a plurality of different light sources.

11. The device according to claim 10, wherein said plurality of light sources have different spectral characteristics such that the spectral composition of the optical radiation emitted from the radiation unit(s) can be adapted by controlling the light sources independently.

12. The device according to any of the preceding claims, wherein the radiation unit comprises one or more elements and/or layers of one or more phosphorizing compounds.

13. The device according to claim 12, further comprising one or more light sources arranged in the radiation unit(s) behind said one or more elements and/or layers of one or more phosphorizing compounds.

14. The device according to any of the preceding claims, wherein said least one radiation unit comprises a light guide for guiding at least a part of said optical radiation.

## Patentansprüche

1. Einrichtung zur nicht-invasiven Licht-Therapie, welche ein oder mehrere Strahlungseinheiten umfasst, die angepasst sind, über mindestens einen Gehörgang optische Strahlung zu einem oder mehreren Gehimstrukturen eines Nutzers zu liefern, **dadurch gekennzeichnet, dass** die Einrichtung angepasst ist, die optische Strahlung von einer Strahlungseinheit mit einem bestimmten Verhältnis einer ersten spektralen, über ein erstes Wellenlängenintervall von 410 nm bis 480 nm integrierten Ausgabeleistung, und einer zweiten spektralen, über ein zweites Wellenlängenintervall von 485 bis 700 nm integrierten Ausgabeleistung, zu emittieren, worin das Verhältnis zwischen 0,45 und 0,65 liegt.

2. Einrichtung nach Anspruch 1, welche weiter ein Gehäuse in optischer und/oder elektrischer Verbindung mit der einen oder den mehreren Strahlungseinheiten und der einen oder mehreren Lichtquellen zur Erzeugung der optischen Strahlung umfasst.

3. Einrichtung nach einem der vorstehenden Ansprüche, welche weiter Mittel zur Anpassung der Intensität der optischen Strahlung umfasst.

4. Einrichtung nach einem der vorstehenden Ansprüche, welche weiter Mittel zur Anpassung der Spektralzusammensetzung der optischen Strahlung umfasst.

5. Einrichtung nach einem der vorstehenden Ansprüche, worin die von einer Strahlungseinheit emittierte optische Strahlung mit einer bestimmten Spektralzusammensetzung eingesetzt wird.

6. Einrichtung nach einem der vorstehenden Ansprüche, worin die von einer Strahlungseinheit emittierte optische Strahlung mit einem oder mehreren der folgenden Parameter eingesetzt wird, die für mehrere bestimmte Wellenlängenintervalle bestimmt sind: Dauer, Intensität, Gesamtleistung, Spektralausgabe und Spektralzusammensetzung.

7. Einrichtung nach einem der vorstehenden Ansprüche, worin das bestimmte Verhältnis zwischen 0,45 und 0,5, oder zwischen 0,5 und 0,55, oder zwischen 0,55 und 0,6 oder zwischen 0,6 und 0,65 liegt.

8. Einrichtung nach einem der vorstehenden Ansprüche, worin das erste Wellenlängenintervall einen Startpunkt von 410 nm, 415 nm, 420 nm, 425 nm, 430 nm oder 435 nm, und einen Endpunkt von zwischen 450 nm, 455 nm, 460 nm, 465 nm, 470 nm, 475 nm oder 480 nm aufweist.

9. Einrichtung nach einem der vorstehenden Ansprüche, worin das zweite Wellenlängen-intervall einen Startpunkt von 485 nm, 490 nm, 500 nm, 505 nm, 510 nm oder 515 nm und einen Endpunkt von 600 nm, 610 nm, 620 nm, 630 nm, 640 nm, 650 nm, 660 nm, 670 nm, 680 nm, 690 nm oder 700 nm aufweist.

10. Einrichtung nach einem der vorstehenden Ansprüche, worin die optische Strahlung von mehreren unterschiedlichen Lichtquellen erzeugt wird.

11. Einrichtung nach Anspruch 10, worin die mehreren Lichtquellen unterschiedliche spektrale Eigenschaften aufweisen, so dass die Spektralzusammensetzung der von den Strahlungseinheit(en) emittierten optischen Strahlung durch unabhängiges Steuern der Lichtquellen angepasst werden kann.

12. Einrichtung nach einem der vorstehenden Ansprüche, worin die Strahlungseinheit ein oder mehrere Elemente und/oder Schichten einer oder mehrerer phosphoreszierender Verbindungen umfasst.

13. Einrichtung nach Anspruch 12, welche weiter ein oder mehrere Lichtquellen umfasst, die in den Strahlungseinheit(en) hinter den ein oder mehreren Elementen und/oder Schichten der einen oder mehreren phosphoreszierenden Verbindungen angeordnet sind.

14. Einrichtung nach einem der vorstehenden Ansprüche, worin die mindestens eine Strahlungseinheit eine Lichtleitung zum Leiten mindestens eines Teils der optischen Strahlung umfasst.

## Revendications

1. Dispositif de luminothérapie non effractive comprenant une ou plusieurs unités de rayonnement conçues pour diriger un rayonnement optique vers une ou plusieurs structures cérébrales neuroanatomiques d'un utilisateur par l'intermédiaire d'au moins un canal auditif, **caractérisé en ce que** le dispositif est conçu pour émettre le rayonnement optique à partir d'une unité de rayonnement avec un rapport prédéfini entre une première puissance de sortie spectrale intégrée sur un premier intervalle de longueur d'onde de 410 nm à 480 nm et une deuxième puissance de sortie spectrale intégrée sur un deuxième intervalle de longueur d'onde de 485 nm à 700 nm, et dans lequel ledit rapport est compris entre 0,45 et 0,65.

2. Dispositif selon la revendication 1, comprenant en outre un logement en connexion optique et/ou électrique avec lesdites une ou plusieurs unités de rayonnement et une ou plusieurs sources de lumière pour générer le rayonnement optique.

3. Dispositif selon l'une quelconque des revendications précédentes, comprenant en outre des moyens pour adapter l'intensité du rayonnement optique.

4. Dispositif selon l'une quelconque des revendications précédentes, comprenant en outre des moyens pour adapter la composition spectrale du rayonnement optique.

5. Dispositif selon l'une quelconque des revendications précédentes, dans lequel le rayonnement optique émis par une unité de rayonnement est appliqué avec une composition spectrale prédéfinie.

6. Dispositif selon l'une quelconque des revendications précédentes, dans lequel le rayonnement optique émis par une unité de rayonnement est appliqué avec un ou plusieurs des paramètres suivants qui sont prédéfinis pour une pluralité d'intervalles de longueur d'onde prédéfinis : une durée, une intensité, une puissance totale, une sortie spectrale et une composition spectrale.

7. Dispositif selon l'une quelconque des revendications précédentes, dans lequel ledit rapport prédéfini est compris entre 0,45 et 0,5, ou entre 0,5 et 0,55, ou entre 0,55 et 0,6, ou entre 0,6 et 0,65.

8. Dispositif selon l'une quelconque des revendications précédentes, dans lequel ledit premier intervalle de longueur d'onde a un point de début à 410 nm, 415 nm, 420 nm, 425 nm, 430 nm ou 435 nm et un point de fin à 450 nm, 455 nm, 460 nm, 465 nm, 470 nm, 475 nm ou 480 nm.

9. Dispositif selon l'une quelconque des revendications précédentes, dans lequel ledit deuxième intervalle de longueur d'onde a un point de début à 485 nm, 490 nm, 500 nm, 505 nm, 510 nm, ou 515 nm et un point de fin à 600 nm, 610 nm, 620 nm, 630 nm, 640 nm, 650 nm, 660 nm, 670 nm, 680 nm, 690 nm ou 700 nm.

10. Dispositif selon l'une quelconque des revendications précédentes, dans lequel le rayonnement optique est généré par une pluralité de sources de lumière différentes.

11. Dispositif selon la revendication 10, dans lequel ladite pluralité de sources de lumière ont différentes caractéristiques spectrales de sorte que la composition spectrale du rayonnement optique émis par l'unité ou les unités de rayonnement peut être adaptée en commandant les sources de lumière de manière indépendante.

12. Dispositif selon l'une quelconque des revendications précédentes, dans lequel l'unité de rayonnement comprend un ou plusieurs éléments et/ou une ou plusieurs couches d'un ou de plusieurs composés phosphorescents.

13. Dispositif selon la revendication 12, comprenant en outre une ou plusieurs sources de lumière agencées dans l'unité ou les unités de rayonnement derrière lesdits un ou plusieurs éléments et/ou lesdites une ou plusieurs couches d'un ou de plusieurs composés phosphorescents.

14. Dispositif selon l'une quelconque des revendications précédentes, dans lequel ladite au moins une unité de rayonnement comprend un guide de lumière pour guider au moins une partie dudit rayonnement optique.
